# EUROPEAN PATENT SPECIFICATION

(11) **EP 3 528 901 B1**
(45) Date of publication and mention of the grant of the patent: **09.12.2020**
(21) Application number: 17780778.1
(22) Date of filing: 13.10.2017
(51) Int. Cl.: A61Q 11/00, A61C 17/00, A61K 8/73, A61K 8/02

(54) **ORAL CARE PARTICLES AND SYSTEM FOR THE ADMINISTRATION THEREOF**
MUNDPFLEGEPARTIKELN UND SYSTEM ZUR DEREN VERABREICHUNG
PARTICULES POUR SOINS BUCCAUX ET SYSTÈME POUR LEUR ADMINISTRATION

(30) Priority: 18.10.2016 EP 16194462
(43) Date of publication of application: 28.08.2019
(73) Proprietor: Koninklijke Philips N.V., 5656 AG Eindhoven (NL)
(72) Inventor: GOTTENBOS, Bart, 5656 AE Eindhoven (NL)
(74) Representative: Marsman, Albert Willem
(86) International application number: PCT/EP2017/076144
(87) International publication number: WO 2018/073106

(56) References cited:
- WO-A1-2016/050573
- US-A1- 2013 108 982
- US-B2- 7 118 376

## Description

### FIELD OF THE INVENTION

The invention is in the field of oral care, and pertains to a system and the system for use in a method for the administration of oral care agents in the form of particles. Notably, the invention pertains to the interdental administration of adhesive gel particles.

### BACKGROUND OF THE INVENTION

The human oral cavity, notably teeth and gums, is generally in need of oral care agents. Think of, e.g., fluoride, remineralization agents, antiplaque agents, anti-tartar agents, anti-gingivitis agents, anti-bacterial agents, and others.

Such agents are generally administered from toothpastes and/or oral rinse liquids. Due to the typical environment of the oral cavity, e.g. having saliva present, a standard difficulty in the art is that active agents from toothpastes and oral rinses are quickly reducing in concentration after their application. Therefore they cannot protect the mouth for long times, and they need therefore to be applied several times daily.

An interesting development in the field is to provide oral care agents in the form of particles. These can be, e.g., solid particles, gel particles, or vesicles. Such particles notably serve to provide a controlled release, e.g., a sustained release, of oral care active agents therefrom. This promises to be an attractive solution to maintain the concentration of oral care agents for longer times or to slow down the decrease in concentration of oral care agents. Particles, notably gel particles, generally have a low volume of solids (typically 1-2%) and therefore can contain a large volume of active formulation.

It is an ongoing challenge, however, to deposit oral care particles in such a way that they are less prone to being spitted out or swallowed. Particularly, it is thereby desired to provide the oral care agents with a better substantivity, i.e., a longer residence time in the oral cavity, than would be naturally given, preferably by being deposited and retained in the interproximal spaces. It is noted that substantivity, such as retention in the interproximal spaces, plays a particular role in view of the non-invasive character of the administration of oral care agents. This is different from injecting a drug into the body, in order to have it taken up in circulation and act systemically rather than locally.

The interproximal space is the area in the mouth that is most prone to oral disease, since bacteria can easily accumulate in these spaces to cause disease. Common diseases like gingivitis and caries are most prevalent in the interproximal area. Delivery of slow release anti-plaque agents in the interproximal area may be able to prevent or reduce such diseases.

Oral care particles, also for controlled release (such as sustained release) would conventionally be delivered from suspensions simply applied by 30s rinsing with 20 to 30ml of a formulation, as is the current practice when using common antimicrobial mouth rinses. However for delivery in the interproximal space this is far from optimal. Particularly, when rinsing, most of the formulation will be spit out, and most of the particles will adhere to other oral surfaces outside the interproximal area. The interproximal areas that are tighter might not even be treated at all. The only available, and only slight, improvement could be by applying excessive amounts of slow release particles. However, the results are still not optimal, if not just marginal. Moreover, applying excessive amounts of slow release particles would be undesirable for economic reasons, since slow release systems are generally more expensive than common mouth rinse ingredients.

JP 2001163768A describes a sustained release preparation for oral care agents. The preparation has a shape that essentially allows it to be inserted into an interdental space. Typically, this is described as a stick-shape, but other shapes (column, cone, plate, columnar, prism, cone, or wedge) are foreseen. The application thereof will generally need to be done manually. Considering the relatively small size of the sticks, this is quite an elaborate task. It would be desired to provide a sustained release preparation for oral care that can be applied using a jetting device, typically releasing air or water under pressure.

US 7,118,376 B2 describes a system for the srategic controlled delivery of materials to the dental surfaces of the intraoral cavity.

### SUMMARY OF THE INVENTION

In order to better address the foregoing desires, the invention, in one aspect, concerns a preparation for the interdental delivery of oral care agents, the preparation comprising one or more oral care agents contained in particles comprising an orally acceptable release matrix, wherein the particles are resiliently deformable and capable of being compressed so as to fit in an interdental space, wherein the particles have a particle size defined by three orthogonally directed dimensions of which a longest dimension is at most 6 mm, a shortest dimension is at least 1 mm, and at least one dimension is in a range of 1.5 to 3 mm.

In another aspect, the invention presents a system for the administration of particles comprising at least one oral care agent to the interproximal spaces of a subject's teeth, the system comprising a preparation as defined above and a pressure device, said pressure device being configured so as to be loaded with particles as comprised in the preparation, and to allow the jetting of said particles into interdental spaces.

In a further aspect, the invention provides the aforementioned preparation for use in a method of applying an oral care agent to the interproximal spaces of a subject's teeth, the method comprising providing particles as contained in the aforementioned preparation, exerting pressure on said particles so as to allow the particles to become sufficiently compressed so as to fit into one or more interproximal spaces, and applying the compressed particles to said one or more interproximal spaces.

### BRIEF DESCRIPTION OF THE DRAWINGS

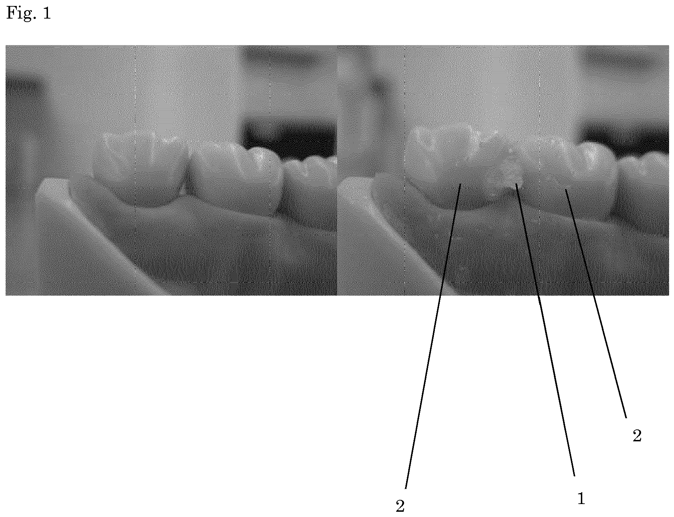

Fig.1 is a photograph showing a dental model, before and after the application of a compressible particle of the invention.

### DETAILED DESCRIPTION OF THE EMBODIMENTS

In a general sense, the invention is based on the judicious insight to introduce oral care agents into interdental spaces by include such agents in resiliently deformable particles that can be compressed so as to fit into said spaces. In the art of dental care agents, it will be understood that particles are typically single, discrete pieces of solid material, e.g. grains or powders. Once a particle of this type has been compressed into an interdental space, the particle's resilience will result in an expanding force. This works, by nature, in a direction opposite from that of the compression and thus into the direction of the surrounding environment. This contributes to the particle being well retained in between teeth, and particularly serves to prevent it from being easily washed away again. As a further advantage, the particles of the present invention can still be removed if desired, by applying a water jet at a force substantially higher than that of the flow of saliva in the mouth, e.g. by high pressure from a water tap.

The terms "interdental" and "interdental space" refer to the space, or gap, between teeth. In the art this is generally also referred to as "interproximal" and "interproximal space."

The particles of the preparation of the invention are capable of being compressed so as to fit in an interdental space. It will be understood that, before being compressed, the particles are thus larger than the gap size of the interdental spaces into which they are to be introduced. It will also be understood that a suitable size of the non-compressed particles is not too far above the desired size after compression, since otherwise too large an extent of compression would be required for the particles to fit in an interdental space.

By way of general guidance, it is noted that typical gaps of interdental spaces are 1 mm to 1.5 mm at their widest point. Accordingly, at least one dimension (such as the width) of the particles generally is greater than 1 mm, preferably greater than 1.5 mm. Considering the space available in the mouth, and the general sensitivity of subjects when feeling foreign objects in their mouth, the longest dimension of the particles desirably is not too large, and generally at most 6 mm, preferably 5 mm or lower. The particles can have different shapes, e.g. spherical, prolate (elongate) spherical, oblate spherical, or irregular variations of such shapes. Accordingly, the particles can have different dimensions in different directions. It is therefore clarified that the particle size is defined with reference to three orthogonally directed dimensions (commonly understood as an x-axis, y-axis, and z-axis). With reference to the aforementioned particle sizes, of these three dimensions the longest dimension is at most 6 mm, and the shortest dimension is at least 1 mm. With reference to the typical interdental space gap size, at least one dimension is in a range of 1.5 to 3 mm. Preferably, in all of the above three dimensions, the lengths are within a range of from 1.5 mm to 2.5 mm.

In an interesting embodiment, the particles have a substantially spherical shape. Thereby the spheres have a diameter that preferably is in the range of from 1.5 mm to 2.5 mm. In another interesting embodiment, the particles have an elongated shape. An advantage hereof is that such particles are capable of filling a larger part of the interdental space. Thereby the longest dimension preferably is 1.5 to 3 times as long as the shortest dimension. A preferred elongated shape is a prolate spherical shape. This shape can have rounded ends or pointed ends. It is preferred for the particles to have a narrowing diameter towards the ends of the prolate spheres, i.e., tending towards pointed ends, as this serves to contribute to the particles staying in place, since such particles will typically be wedged into the interdental space, in addition to the effect of compression and subsequent expansion of the compressible and resiliently deformable particles used in the system of the invention.

The particles used in the system of the invention comprise an orally acceptable release matrix. The invention makes use of particles having the property of being resiliently deformable, and can be compressed. It will be understood that this property can be provided by many materials, since elastomeric materials, such as rubber materials, are well known. To a skilled chemist, it is generally possible to adapt the composition of polymeric materials, hence also in the event of elastomers, to the desired releasability of an agent, here an oral care agent, contained therein. In general this involves adapting the hydrophilic or hydrophobic character of polymer main chains, side chains, functional groups, or a combination thereof, as needed for the type of agent concerned.

Preferably, in the field of interdental delivery of oral care agents, the release matrix is an edible hydrogel. Preferred hydrogels are made from a material selected from the group consisting of chitosan, alginate, carrageenan, xanthan gum, and mixtures thereof, generally by crosslinking such materials. These hydrogels are well-known in the field. Preferably, the hydrogel used in the system of the invention is mucoadhesive, a further preferred example thereof being gelled chitosan. It is also possible for the particles to be coated with an oral care acceptable adhesive layer. The particles can hydrated, but can also be in a dry form. Wetting agents different from water can also be used, as is known for anhydrous gels.

Mucoadhesive materials (which are a form of bioadhesive materials capable of adhering to mucosa) are well-known in the field, e.g., from widely available knowledge on mucoadhesive drug delivery systems.

The skilled person is well able to produce hydrogels that are compressible and resiliently deformable. This is generally accomplished by a method providing an aqueous solution of the gel forming component, such as chitosan, and mixing the solution with an aqueous crosslinker solution comprising a crosslinking agent, such as a tribasic or tetrabasic salt, or a mixture thereof.

Typically, chitosan is produced in molecular weights ranging from 3800 to 20,000 Daltons. In order to produce resiliently deformable hydrogels, it is preferred to use relatively high molecular weight chitosan, such as above 10,000 Daltons, such as 15,000 to 20,000 Daltons. Similar considerations hold for other gel-forming components. Also, it is preferred to apply a relatively hugh degree of crosslinking.

In a preferred embodiment, the compressibility of the particles is enhanced by the presence of a plurality of voids. Such voids can take the form of entrained air pockets (air pockets dispersed through the material), such as in a foam, or a typically more open structure comprising pores (such as in a sponge like structure). It will be understood that voids can be filled air, or with an inert gas such as nitrogen. Gas (air) bubbles can be entrained during processing just before a crosslinking / gelation step in various ways. This can be by mixing air into the gelling liquid and preferably by applying a bubble generator, e.g. by providing gas (such as nitrogen or air) via a tube with small holes. The bubbles then become mixed with the liquid, resulting in drops or jets that are crosslinked and which comprise voids.

The particles contain one or more oral care agents. Particularly, these agents are agents for which it is desired to be administered into the interdental (interproximal) spaces. Preferred agents are selected from the group consisting of antiplaque agents, anti-tartar agents, anti-gingivitis agents, anti-caries agents, anti-bacterial agents, anti-periodontitis agents, mineralization agents, bleaching agents, and combinations thereof. With reference to the advantage of being well-retained in interdental spaces, and particularly for the sustained release of such agents, the preferred agents for use in the present invention are anti-bacterial agents. These include, for example, phenolics and salicylamides, and sources of certain metal ions such as zinc, copper, silver and stannous ions, for example in salt form such as zinc , copper and stannous chloride, and silver nitrate. These are present in art-known small quantities when used. Typical oral care agents in common usage are chlorhexidine digluconate, cetylpyridinium chloride, stannous fluoride, sodium fluoride, hydrogen peroxide, zinc citrate, benzethonium chloride, zinc lactate, phenolic compounds (e.g., thymol, menthol, eucalyptol), triclosan, herbal extracts (e.g. sanguinarine).

The preparation of the invention can be administered into the interproximal spaces of a subject's teeth, by applying a force on a particle so as to compress and/or deform the particle sufficiently for it to enter the interdental space. This can be accomplished by exerting a force on each individual particle, and applying such particle in an interdental space. This can be repeated to apply further particles to further interdental spaces. The pressure can be applied manually, and the administration can also be done manually. Preferably, however, both exerting the pressure and administering the particle, is done by means of a suitable pressure device, typically jetting a particle by force into the oral cavity, at an interdental space of choice. In the event of using a pressure device, administering the preparation can also be accomplished by exerting a force on a plurality of particles, and jetting a plurality of particles into the oral cavity, generally directed at interdental spaces. This is a quicker and easier operation, but it will also lead to loss of excess particles. It is preferred to jet one particle at the time.

Generally, the particles will be transported into a pressure device from a particle storage container, either as a separate container, or as a storage compartment associated with or fully integrated with a jetting device. Transportation can be done manually or mechanically, e.g. compressing the particles mechanically and transporting them by means of a manually or mechanically operated plunger. Preferably, transporting the particles is done by means of a flow of gas (particularly air) or liquid (particularly water). A combination of the foregoing techniques is also possible. E.g., the particles can be transported and delivered into an applicator device by an air pulse, and compressed between the teeth by a mechanical pushing mechanism in the tip of the applicator.

The invention also pertains to a system for the administration of particles as defined hereinabove, to the interproximal spaces of a subject's teeth. The system comprises as its main components a preparation as defined above, and a pressure device. The pressure device is configured so as to be loaded with particles as comprised in the preparation, and to allow the jetting of said particles into interproximal spaces. The pressure device preferably is a fluid jet generator, more preferably an oral irrigator.

Jet generating devices such as oral irrigators have been applied in the art for purposes such as interdental cleaning (flossing). For this purpose, these devices, also known as interdental cleaners, have turned out to be greatly effective. Effective cleaning of the dental interproximal space would normally be at odds with delivering substances to said space. Rather, the interdental cleaning serves the purpose of removing substances (notably dental plaque) contained in the interproximal spaces. In accordance with the foregoing, WO 2013/093798 describes spray velocities running from 10 m/s to 300 m/s, e.g. 50 m/s. In the present invention, such generating devices are also used for the purpose of delivering substances to be retained in the interproximal space. This can be accomplished e.g. as described in WO 2016/050573.

The introduction of the particles comprising the oral care agent as a fluid jet, can be done by means of any device or unit capable of generating a fluid jet. E.g., in one embodiment, this can be a nozzle which is fed with the liquid under a pressure sufficient to generate the desired jet speed. In the system for use in the method of the invention, the nozzle is directed to the mouth, such as to introduce the fluid jet into the oral cavity. Preferably, the device is adapted so as to enable directly reaching the interproximal spaces. To this end, the nozzle can take the form of a flexible or rigid tube, having a tip the dimensions of which allow a sufficient degree of precision at directing the fluid jet expelled therefrom to a desired location within the oral cavity, preferably such that localization directly into the interproximal spaces is possible. Alternatively, a syringe can be applied. An oral irrigator typically has a single nozzle, as the device is intended for the separate and precise cleaning of individual location, such as interproximal spaces, in the oral cavity.

In an interesting embodiment, a pulsed jet delivery of approximately 0.1ml is provided by a plunger pump, for example driven by a pre-loaded spring. After each shot the syringe can be refilled from a larger suspension reservoir (as a container unit), while loading the spring.

The skilled person will be aware of devices and nozzles that are suitable for the aforementioned purposes. Particularly suitable types of devices are the above-mentioned oral irrigators, including interdental cleaners and liquid-assisted flossing devices.

An oral irrigator, such as an interdental cleaner, typically comprises a source of liquid; a system for moving a selected amount of liquid from the source thereof into a liquid pathway; a driving unit such as a pump or a source of pressurized gas, or a combination thereof; and a control arrangement for releasing a selected amount of gas into contact with the liquid, resulting in liquid being propelled out of a nozzle portion of the cleaner. Suitable devices are described, *inter alia*, in WO 2010/055433, WO 2010/055434, WO 2008/012707, WO 2014/068431.

The oral irrigators to which the invention applies, particularly function on the basis of a single nozzle being used at a time. These devices are adapted to be used for applying a liquid jet to each individual interproximal space separately. Also, it is to be understood that an oral irrigator is a device that, upon use, is held in the hand while kept for the most part outside of the mouth, or at least not in touch with the teeth. This is opposed to devices such as mouthpieces that are essentially to be placed over teeth, and kept in the mouth during their use. The fundamental distinction between such devices is known in the art, see e.g. the background section in US 2013/236851.

The fluid jet-generating devices suitable for use in the invention can be adapted to provide continuous jets, or separate shots of jets, or both. For the use of the present invention it is preferred if single shots can be provided. This would allow a greater precision in administering the above described particles to the various interproximal spaces one at a time. Such preferred devices are well-known in the art.

Preferably, the application device is provided with a container for retaining the particles prior to their compression and delivery. E.g., the device can be provided with a reclosable aperture allowing a container to be attached as a separate module. Such a module is preferably replaceable so as to facilitate refilling the device with fresh particles. Also, such modules can then be exchanged so as to administer different particles having different oral care agents.

In an interesting embodiment, a container for particle storage is configured to be in fluid communication with the nozzle of a fluid jet generator. The particles are then preferably held in an aqueous fluid suspension, and will be supplied in this container in fluidic connection to the nozzle, where at each air pulse a particle is loaded into the nozzle. The connection is preferably provided with a venturi section, as this will result in an underpressure that acts to load a particle bead from the container. The nozzle is preferably configured to be wider than the particles, so it can easily transport the beads to the teeth. Preferably the nozzle is only slightly wider, e.g. 5-25% such as 10-20% wider than the largest dimension of the particles.

The fluid jet generator will be as discussed above in respect of oral irrigators, such as interdental cleaners. The container unit can be part of a fluid jet generating device, but it can also be a separate unit, whereby fluid communication is provided between an outlet of the container and an inlet of the jet generator. Such fluid communication can be provided by suitable tubes or flow lines, with suitable fixation of one to the other. Also, the jet generator unit can be provided with a holder for a cartridge, whereby the cartridge serves as a container for the liquid. The source of liquid, with which the container unit is in fluid communication, can be present in the container itself, viz. as a suspension comprising the particles. The source of liquid can also be provided from one container, and the particles from another. The source of liquid can also be an external source, to which the system of the invention can be hooked-up, or with which the system of the invention can be connected, so as to provide the required fluid communication between the container unit for the particles, and the source of liquid.

The system of the invention preferably further comprises a dental appliance for cleaning teeth, selected from the group consisting of electric toothbrushes, electric flossing devices, and combinations thereof. Such dental appliances can be provided for various functions. This typically refers to a toothbrush, preferably an electrical toothbrush, more preferably a sonic power toothbrush having a vibrating brush head.

If not already provided by the jetting system of the invention itself, an electric flossing device, as is possibly comprised in the system of the invention, refers to such devices that serve to clean the interdental spaces generally by spraying air, by spraying liquid, or a combination thereof.

It is to be understood that the system can comprise its various parts as separate components, not packaged or provided together.

Particularly, the container holding the particles comprising at least one oral care agent can well be provided as a separate entity, e.g., in the form of a bottle or tube holding the composition (comparable to a bottle of mouthwash or a toothpaste tube). The container can also be attached to the delivery device, particularly as a cartridge adapted for such an attachment, e.g. to an electric toothbrush (designed with a separate fluid delivery system), flossing device or an oral irrigator, such as a Philips Sonicare AirFloss or Philips Sonicare Toothbrush, with a delivery pump.

In an interesting further embodiment, the system according to the invention comprises a power module and one or more dental appliance heads that can be removably attached to said power module. This typically refers to having an electric toothbrush and/or an electric flossing device, both preferably provided as functional modules in the form of dental appliance heads.

While the invention has been illustrated and described in detail in the drawings and foregoing description, such illustration and description are to be considered illustrative or exemplary and not restrictive; the invention is not limited to the disclosed embodiments.

For example, it is possible to operate the invention in an embodiment wherein a fluid jet generator is provided with two or more containers, each container holding different oral care agents.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain features of the invention are recited in mutually different dependent claims does not indicate that a combination of these features cannot be used to advantage. Any reference signs in the claims should not be construed as limiting the scope.

In sum, we hereby disclose a preparation for the interdental delivery of oral care agent. The preparation comprising one or more oral care agents contained in particles comprising an orally acceptable release matrix, wherein the particles are resiliently deformable and capable of being compressed so as to fit in an interdental space. The particles can thus be provided in a size slightly larger than that of an interdental space. By compression they can be inserted, particularly jetted, into interdental spaces, and their tendency to thereupon expand again provides a force retaining them in the space where they were applied, without being prone to washing away by saliva.

The invention will be further explained hereinafter with reference to the examples and figures. These illustrate the invention, but do not limit it.

### Example 1

Particles in the form of compressible beads were made of chitosan hydrogel. A 2 wt.% high viscous chitosan solution in 0.1M HCl + 0.1% CPC was turned into a bubble foam using a mixer (Philips Sonicare toothbrush) for 4 minutes. The chitosan foam (2ml) was dripped into 20ml 0.1M NaOH stirring at intermediate rotation speed to crosslink the drops. Although chitosan gel without bubbles shows elastic behavior, the bubbles increase the compressibility of the beads. These beads can be characterized as a hydrogel foam. The beads were taken out the NaOH solution and placed in a slightly acidic 2mM NaH₂PO₄ solution.

### Example 2

Interproximal delivery of particles was tested in an anatomical model of the interproximal space. A bead of example 1 was delivery to a model interdental space (Typodont Frasaco AG3) using an "AirFloss ultra" without water (air pulse only). The bead was placed in the nozzle, the nozzle placed against the interdental space, and a single air pulse was given to deliver the bead. As shown in Figure 1 the bead (1) was successfully delivered in the interdental space between two teeth (2). On the left hand side, the figure shows the dental model before application of the bead, and on the right hand side with the bead applied. Moderate washing under a water tap did not remove the bead, but a water tap with higher pressure did remove the bead, showing the bead can still be dislodged if desired.

## Claims

1. A preparation for the interdental delivery of oral care agents, the preparation comprising one or more oral care agents contained in particles comprising an orally acceptable release matrix, wherein the particles are resiliently deformable and capable of being compressed so as to fit in an interdental space, wherein the particles have a particle size defined by three orthogonally directed dimensions of which a longest dimension is at most 6 mm, a shortest dimension is at least 1 mm, and at least one dimension is in a range of 1.5 to 3 mm.

2. A preparation according to claim 1, wherein the particles comprise a plurality of internal voids.

3. A preparation according to claim 2, wherein the voids comprise dispersed air pockets.

4. A preparation according to any one of the preceding claims, wherein the release matrix is an edible hydrogel, particularly made from a material selected from the group consisting of chitosan, alginate, carrageenan, xanthan gum, and mixtures thereof.

5. A preparation according to any one of the preceding claims, wherein all three dimensions are within a range of from 1.5 mm to 2.5 mm.

6. A preparation according to claim 5, wherein the particles have a substantially spherical shape.

7. A preparation according to any one of the claims 1-4, wherein the longest dimension is 1.5 to 3 times as long as the shortest dimension.

8. A preparation according to claim 7, wherein the particles have a substantially prolate spherical shape.

9. A preparation according to any one of the preceding claims, wherein the particles are mucoadhesive, preferably comprising gelled chitosan.

10. A preparation according to any one of the preceding claims, wherein the one or more oral care agents are selected from the group consisting of antiplaque agents, anti-tartar agents, anti-gingivitis agents, anti-caries agents, anti-bacterial agents, anti-periodontitis agents, mineralization agents, bleaching agents, and combinations thereof.

11. A system for the administration of particles comprising at least one oral care agent to the interproximal spaces of a subject's teeth, the system comprising a preparation according to any one of the preceding claims and a pressure device, said pressure device being configured so as to be loaded with particles as comprised in the preparation, and to allow the jetting of said particles into interproximal spaces.

12. A system according to claim 11, wherein the pressure device is fluid jet generator, preferably an oral irrigator.

13. A system according to any one of the claims 11-12, further comprising a dental appliance for cleaning teeth selected from the group consisting of electric toothbrushes, electric flossing devices, oral irrigators, and combinations thereof.

14. Particles as defined in any one of the claims 1-10 for use in a method of applying an oral care agent to the interproximal spaces of a subject's teeth, the method comprising providing particles as defined in any one of the claims 1-10, exerting pressure on said particles so as to allow the particles to become sufficiently compressed so as to fit into one or more interproximal spaces, and applying the compressed particles.

## Patentansprüche

1. Ein Präparat für die interdentale Verabreichung von Mundpflegemitteln, wobei das Präparat mindestens ein Mundpflegemittel umfasst, das in Partikel eingeschlossen ist, die eine orale Freisetzungsmatrix umfassen, und wobei die Partikel elastisch verformbar sind und komprimiert werden können, um sich für den Interdentalraum zu eignen, und wobei die Partikel eine Teilchengröße aufweisen, die durch drei orthogonal ausgerichtete Abmessungen definiert ist, von denen die längste Abmessung bei höchstens 6 mm, die kürzeste Abmessung bei mindestens 1 mm und mindestens eine Abmessung in einem Bereich von 1,5 bis 3 mm liegt.

2. Ein Präparat gemäß Anspruch 1, wobei die Partikel mehrere innere Hohlräume aufweisen.

3. Ein Präparat gemäß Anspruch 2, wobei die Hohlräume verteilte Lufttaschen umfassen.

4. Ein Präparat gemäß einer der vorherigen Ansprüche, wobei es sich bei der Freisetzungsmatrix um ein essbares Hydrogel handelt, das hauptsächlich aus einem Material besteht, das aus der folgenden Gruppe ausgewählt wurde: Chitosan, Alginat, Carrageen, Xanthangummi und Mischungen hiervon.

5. Ein Präparat gemäß einer der vorherigen Ansprüche, wobei alle drei Abmessungen in einem Bereich von 1,5 mm bis 2,5 mm liegen.

6. Ein Präparat gemäß Anspruch 5, wobei die Partikel im wesentlichen eine Kugelform aufweisen.

7. Ein Präparat gemäß einer der Ansprüche 1 bis 4, wobei die längste Abmessung 1,5 bis 3 mal so lang wie die kürzeste Abmessung ist.

8. Ein Präparat gemäß Anspruch 7, wobei die Partikel eine im Wesentlichen längliche Kugelform aufweisen.

9. Ein Präparat gemäß einer der vorherigen Ansprüche, wobei die Partikel mukös haftend sind und vorzugsweise geliertes Chitosan enthalten.

10. Ein Präparat gemäß einer der vorherigen Ansprüche, wobei das mindestens eine Mundpflegemittel aus der folgenden Gruppe ausgewählt wird: Mittel gegen Plaque, Mittel gegen Zahnstein, Mittel gegen Zahnfleischentzündung, Mittel gegen Karies, antibakterielle Mittel, Mittel gegen Parodontose, Mineralisierungsmittel, Bleichmittel und Kombinationen hiervon.

11. Ein System zum Verabreichen von Partikeln, die mindestens ein Mundpflegemittel enthalten, in den Zahnzwischenräumen einer Person, wobei das System ein Präparat gemäß einer der vorherigen Ansprüche sowie ein Druckgerät umfasst, und wobei das Druckgerät mit den im Präparat enthaltenen Partikeln befüllt werden kann, um diese in die Zahnzwischenräume einzuführen.

12. Ein System gemäß Anspruch 11, wobei es sich beim Druckgerät um einen Flüssigkeitsstrahl-Generator und vorzugsweise eine Munddusche handelt.

13. Ein System gemäß einer der Ansprüche 11 und 12, das zudem ein Dentalgerät für die Zahnreinigung umfasst, das aus der folgenden Gruppe ausgewählt wird: elektrische Zahnbürste, elektrische Zahnseidevorrichtung, Munddusche und Kombinationen hiervon.

14. Partikel gemäß einer der Ansprüche 1 bis 10 Partikel zum Aufbringen eines Mundpflegemittels auf die Zahnzwischenräume einer Person, wobei die Methode das Bereitstellen von Partikeln gemäß einer der Ansprüche 1 bis 10 umfasst, das Ausüben von Druck auf die Partikel, damit diese ausreichend komprimiert werden, sodass sie in einen oder mehrere Zahnzwischenräume passen, sowie das Aufbringen der komprimierten Partikel umfasst.

## Revendications

1. Préparation destinée à l'administration interdentaire des agents de soin bucco-dentaire, ladite préparation comprenant au moins un agent de soin bucco-dentaire contenu dans des particules comprenant une matrice de libération oralement acceptable, dans laquelle les particules sont déformables de manière élastique et apte à la compression de manière à s'ajuster dans un espace interdentaire, dans laquelle les particules présentent une taille de particule définie par trois dimensions orientées orthogonalement dont une dimension la plus longue est au plus 6 mm, une dimension la plus courte est au moins 1 mm et au moins une dimension est dans la plage comprise entre 1,5 et 3mm.

2. Préparation selon la revendication 1, dans laquelle les particules comprennent une pluralité de vides internes.

3. Préparation selon la revendication 2, dans laquelle les vides comprennent des poches d'air dispersées.

4. Préparation selon l'une quelconque des revendications précédentes, dans laquelle la matrice de libération est un hydrogel comestible, en particulier fabriqué à partir d'une matière choisi dans le groupe constitué par le chitosane, l'alginate, le carrag-hénane, la gomme de xanthane et leurs mélanges.

5. Préparation selon l'une quelconque des revendications précédentes, dans laquelle les trois dimensions sont dans une plage comprise entre 1,5 mm et 2,5 mm.

6. Préparation selon la revendication 5, dans laquelle les particules présentent une forme sensiblement sphérique.

7. Préparation selon l'une quelconque des revendications 1 à 4, dans laquelle la dimension la plus longue est de 1,5 à 3 fois supérieure à la dimension la plus courte.

8. Préparation selon la revendication 7, dans laquelle les particules présentent une forme sphérique sensiblement allongée.

9. Préparation selon l'une quelconque des revendications précédentes, dans laquelle les particules sont mucoadhésives, de préférence comprenant le chitosane gélifié.

10. Préparation selon l'une quelconque des revendications précédentes, dans laquelle l'au moins un agent de soins bucco-dentaires est choisi dans le groupe constitué par des agents anti-plaque, des agents anti-tartre, des agents anti-gingivite, des agents anti-carie, des agents anti-bactériens, des agents anti-parodontite, des agents de minéralisation, des agents de blanchiment, et combinaisons de ceux-ci.

11. Système destiné à l'administration des particules comprenant au moins un agent de soins bucco-dentaires dans les espaces interproximaux des dents d'un sujet, ledit système comprenant une préparation selon l'une quelconque des revendications précédentes et un dispositif de pression, ledit dispositif de pression étant conçu de manière à être chargé de particules telles que comprises dans la préparation, et pour permettre un jet desdites particules dans les espaces interproximaux.

12. Système selon la revendication 11, dans lequel le dispositif de pression est un générateur de jet de fluide, de préférence un irrigateur oral.

13. Système selon l'une quelconque des revendications 11 à 12, comprenant en outre un appareil dentaire pour nettoyer les dents choisi dans le groupe constitué par les brosses à dents électriques, les dispositifs de lustrage électriques, les irrigateurs buccaux et leurs combinaisons.

14. Particules telles que définies dans l'une quelconque des revendications 1 à 10 destinées à être utilisées dans un procédé d'application d'un agent de soins bucco-dentaires aux espaces interproximaux des dents d'un sujet, ledit procédé comprenant la fourniture de particules telles que définies dans l'une quelconque des revendications 1 à 10, par exercice d'une pression sur lesdites particules de manière à permettre aux particules de se comprimer suffisamment de manière à s'ajuster dans au moins un espace interproximal et l'application des particules compressées.
